Europäisches Patentamt

European Patent Office

Office européen des brevets

(11) Publication number: **0 258 356 B1**

## EUROPEAN PATENT SPECIFICATION

(45) Date of publication of patent specification: **22.09.93**  (51) Int. Cl.5: **A61K 9/72, A61K 9/16**

(21) Application number: **87901468.6**

(22) Date of filing: **27.02.87**

(86) International application number:
**PCT/EP87/00118**

(87) International publication number:
**WO 87/05213 (11.09.87 87/20)**

(54) **NEW PHARMACEUTICAL COMPOSITIONS FOR INHALATION.**

(30) Priority: **04.03.86 IT 1962586**

(43) Date of publication of application:
**09.03.88 Bulletin 88/10**

(45) Publication of the grant of the patent:
**22.09.93 Bulletin 93/38**

(84) Designated Contracting States:
**AT BE CH DE FR GB IT LI LU NL SE**

(56) References cited:
**DE-A- 3 012 136**
**US-A- 3 957 965**

**LEHRBUCH DER PHARMAZEUTISCHEN TECH-NOLOGIE BY VOIGT, 5TH ED., 1980, P. 178-179**

**Hackh's Chem. Dict., 4th Ed., 1972, p. 174**

**Römpps Chemie-Lexikon, 8th Ed., 1988, p. 1539**

(73) Proprietor: **CHIESI FARMACEUTICI S.p.A.**
**Via Palermo, 26/A**
**I-43100 Parma(IT)**

(72) Inventor: **CHIESI, Paolo**
**Via Palermo, 26/A**
**I-43100 Parma(IT)**
Inventor: **PAVESI, Luciana**
**Via Palermo, 26/A**
**I-43100 Parma(IT)**

(74) Representative: **Minoja, Fabrizio**
**Studio Consulenza Brevettuale Via Rossini, 8**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

EP 0 258 356 B1

**Description**

The present invention relates to a new excipient which may be used in pharmaceutical technology for the preparation of pharmaceutical compositions, in particular powders for inhalation, and to methods for the preparation thereof.

The administration of drugs by aerosol is the main component of the treatment of many patients suffering from respiratory disorders with spastic complications.

The action of the drug is substantially accelerated and prolonged if it is inhaled; since the drug acts directly on the target organ much smaller quantities of the active principle may be used, thereby reducing side effects to a minimum.

The patient must synchronise his breathing correctly with the emission of the spray if pressurised aerosols or like devices are to be successfully used.

Many patients, particularly children, old people, weak patients etc. are not, however, able to use these devices correctly.

The ideal inhaler should enable the administration of uniform and sufficient quantities of the drug with a minimum effort on the part of the patient.

The direct use of powder substances in devices designed for the inhalation of these powders provides a viable alternative in the administration of bronchopulmonary drugs.

The major advantage of powder inhalers is that the active principle is released simultaneously with the act of inhalation by the patient, since the powder mist formed from the particles of the active formulation is generated by the flow of air which is created by inhalation. In practice, the apparatus itself synchronises the two actions.

The operation of all powder inhalation devices depends on the creation of a turbulent air flow designed to disperse the particles of the drug powder in a mist suitable for inhalation.

There are two basic types of such devices which differ in terms of the way in wich the powder is distributed and supplied:

I. Inhalers for the administration of single doses predetermined mechanically by dividing the dosage into capsules. Such devices may be used repeatedly.

II. Inhalers of the dosage metering type for complete treatment cycles, which contain appropriate quantities of active principle, whose unit dose is distributed in a practical and rapid way at the time of use on the basis of the prescribed dosage, enabling, if necessary, the administration of two doses simultaneously.

The supply device is finished at the end of the treatment cycle.

As a result of the relatively complicated construction of inhalers of type I, which are currently in use, patients whose ability to use their hands is impaired find them comparatively difficult to handle.

The fact that the dose of drug to be inhaled is contained in a gelatine capsule also entails major drawbacks:

- each time the drug is to be taken, the patient must insert a capsule in the device and, when the drug has been taken, has to empty and clean capsule and powder residues from the device;
- in many cases the capsule fails to open at the first attempt and the patient has to try a number of times;
- the capsule often fails to empty completely with the result that the patient does not take a uniform drug dosage.

An apparatus of type II whose functional and practical features make it advantageous and convenient to use for the administration of drugs in the form of powders, is disclosed in British Patent Specification 2 041 763 B.

The apparatus essentially comprises the following components:

1. a mouthpiece which rotates freely on a main body;

2. a main body which, in turn, bounds:

2.1 a chamber containing the drug whose volume is such as to contain a quantity of the drug sufficient for a complete treatment cycle;

2.2 a metering mechanism on the base of the storage chamber which, following a rotation through 180°, supplies an exact quantity of substance equivalent to a dose of the drug;

2.3 a distributor system which causes the dose of drug supplied to fall, during rotation, into a cavity provided in the central body of the apparatus;

2.4 the above cavity, which communicates at the top with the mouthpiece chamber and at the bottom with a ventilation chamber located in the space between the dose collection plate and the base baffle of the body itself. The ventilation chamber takes air from two symmetrical apertures which pass through the

2

side walls of the tank.

When the patient wishes to use the apparatus, he rotates the nozzle through 180° on the central body: this position may be visually located by lining up the two arrows marked on the container and the mouthpiece at which point a catch engages.

After the dose has been metered out in this way, the patient, by breathing in from the mouthpiece, generates a flow of air into the lateral aerators.

The powder, taken up by the flow of air, is caused to emerge from the central cavity and inhaled directly at the time of breathing in.

If apparatus of this type is to operate correctly, the powders to be inhaled must have special properties and features.

Exact metering and the accuracy of the prescribed dosage depend, for the entire duration of use of the apparatus, on the correct operation of the mechanical metering mechanism which is an essential component of the system.

The mechanical functions of the device and the technical properties of the content must therefore be accurately matched.

The active doses of drugs used in the treatment of respiratory infections are generally in the range of a milligram or fractions thereof. For this reason, if a uniform dosage is to be obtained, the weight of individual doses must be increased to values which enable reliable metering by diluting the active principle with a suitable inert excipient whose particle size has been monitored such that it does not penetrate into the deeper sections of the respiratory tract.

DE 3 012 136 discloses a microfine vehicle coated with a soluble binder for the preparation of a granulate.

The British Patent Specification 1 242 211 in the name of Fisons discloses a pharmaceutical composition in the form of a powder, formed by a mixture of micronised drug substance and a pharmaceutically acceptable water-soluble excipient with a predetermined particle size distribution.

Lactose is disclosed as the preferred excipient.

Pharmaceutical compositions in powder form of this type have not been found to be particularly advantageous for inhalation devices of the type disclosed in the British Patent Publication 2 041 763. Our experience shows that the powder metered out may penetrate, at the time of rotation to distribute the dose to be inhaled, into the mechanism of the apparatus and, when this powder is composed of micronised active principle diluted in conventional microcrystalline excipients, tends, after a certain number of metering operations, to inhibit the free rotation of the mouthpiece on the central body by means of which the drug is actually metered out. If the rotation in incomplete as a result of the resistance which is encountered, there may, towards the end of treatment, be a decrease in the unit quantity of composition supplied and therefore a decrease in the unit quantity of active principle.

It has now been discovered, and this is an object of the present invention, that this drawback may be remedied and that the properties of pharmaceutical compositions in powder form which may be administered by inhalation using a device of the type disclosed in the British Patent Publication 2 041 763 may be substantially improved if the active principle is mixed with a new type of excipient obtained by specific preparation techniques and provided with specific and special physical properties.

This new excipient, hereafter called a "conglomerate", takes the form of microgranules of a solid water-soluble vehicle such as lactose, xylitol, arabinose, dextran, mannitol or the like and a suitable lubricant such as sodium benzoate, magnesium stearate, colloidal silica, hydrogenated oils, etc., conglomerated in the microgranule.

The conglomerate of the invention may be prepared by adding the lubricant to an aqueous solution of a portion of the solid diluent and the mixture obtained in this way is subsequently used for the granulation of the remainder of the solid diluent and is finally sifted through a 0.03 mm mesh to separate particles of less than 30 $\mu$m from the desired fraction.

The vehicle may, in turn, be constituted by a mixture of powders: a mixture of lactose with 0.5 to 30% by weight of saccharose has been found to be particularly advantageous.

Granules of the type described, formed by a crushed solid excipient conglomerated with a lubricant, have not been disclosed up to now.

Their use as excipients in pharmaceutical compositions in powder form for inhalation has proved to be particularly advantageous since the flow properties of the powder and its self-lubricating properties are considerably improved.

In the specific case of use of the inhaler disclosed in the British Patent Publication 2 041 763, we have found that, there is in fact a self-lubricating effect on the metering mechanism.

As a result of this:

a) the apparatus is less resistant to rotation;

b) the weight of the composition supplied is more uniform;

c) the amount of active principle supplied during each distribution operation is more uniform.

As regards the simple physical mixture, the agglomeration of the lubricant with the excipient also has the advantage of preventing lubricant particles which are too small from being inhaled and penetrating into the lungs.

Among the crushed solid excipients which are possible, latose (possibly with the addition of saccharose) and xylitol are preferred, the latter being particularly advantageous since it remedies the possible problem of dental caries.

Once the conglomerate has been prepared, the pharmaceutical composition may be prepared, using appropriate techniques, by mixing the conglomerate and the active principle, in a suitable powder mixer, until they are homogeneous, the active principle being in the form of a micronised powder preferably such that at least 90% of the particles are between 0.1 and 10$\mu$m in size.

The quantity of conglomerate with respect to the active principle will obviously vary as a function of the dosage of the latter in terms of its degree of activity.

The compositions obtained in this way are metered into inhaler devices.

The formulations may contain a wide variety of drugs which may be administered by inhalation and in particular drugs used in the treatment of bronchopulmonary disorders. Specific examples of substances which may be used in the preparation of compositions of the present invention are drugs having an anti-histamine and anti-allergic action such as sodium cromoglycate and ketotifen; anticholinergics such as ipratropium bromide, oxytropium bromide, thiazinamide chloride; sympathomimetic amines such as terbutaline, salbutamol, clenbuterol, pirbuterol, reproterol, procaterol and fenoterol; corticostercids such as beclomethasone dipropionate, flunisolide, budesonide; mucolytics such as ambroxol and their combinations.

The present invention also relates to a powder inhaler apparatus of the type disclosed in the British Patent Specification 2 041 763 B filled with appropriate quantities of a pharmaceutical composition of the type of the present invention.

The invention is described in further detail in the following examples.

EXAMPLE 1

Preparation of microgranules of the new vehicle (conglomerate)

1A. Composition:

| Lactose F.U. (Italian Pharmacopoeia) | 1000 g |
|---|---|
| Magnesium stearate | 10 g |
| Purified water | 105 g |

A 30% (p/p) aqueous solution of lactose was prepared by dissolving 45 g of lactose F.U. in 105 g of purified water and heating it slightly. When the solution was clear, the magnesium stearate was added and mixed until it was homogeneously dispersed; the suspension was quickly poured into the remaining lactose F.U. (955 g) and mixed. The mixture was passed through a 2 mm mesh and placed in an oven at 55°C. When the mixture was dry, it was granulated through a 1 mm and then a 0.212 mesh and the product was sifted through a 0.03 mm mesh eliminating the < 30$\mu$m particle fraction.

1B. Composition:

| Lactose F.U. | 1000 g |
|---|---|
| Sodium benzoate | 20 g |
| Purified water | 105 g |

A 30% (p/p) aqueous solution of lactose was prepared by dissolving 45 g of lactose F.U. in 105 g of purified water and heating it slightly. When the solution was clear, the sodium benzoate was added and the mixture was stirred until the sodium benzoate was completely dissolved; the solution obtained in this way was quickly poured onto the remaining lactose F.U. (955 g) and mixed. The mixture was passed through a 2 mm mesh and placed in an oven at 55°C. When the mixture was dry, it was granulated through a 1 mm and then a 0.212 mesh. The product was then sifted through a 0.03 mm mesh eliminating the < 30$\mu$m particle fraction.

1C. Composition:

| Lactose F.U. | 955 g |
|---|---|
| Saccharose | 45 g |
| Magnesium stearate | 10 g |
| Purified water | 105 g |

A 30% (p/p) aqueous solution of saccharose was prepared by dissolving 45 g of saccharose in 105 g of purified water and heating it slightly. When the solution was clear, the magnesium stearate was added and mixed until it was homogeneously dispersed; the suspension was quickly poured onto the lactose F.U. (955 g) and mixed. Preparation was continued in the way described under Examples 1A and 1B.

1D. Composition:

| Xylitol | 1000 g |
|---|---|
| Sodium benzoate | 10 g |
| Purified water | 60 g |

A 40% (p/p) aqueous solution of xylitol was prepared by dissolving 40 g of xylitol in 60 g of purified water and heating it slightly. When the solution was clear, the sodium benzoate was added and the mixture was stirred until the sodium benzoate was completely dissolved; the solution obtained in this way was used to granulate the remaining xylitol (960 g) in a fluidized bed. The product collected was passed through a 1 mm and then a 0. 212 mm mesh. The product was then sifted through a 0.03 mm mesh eliminating the < 30$\mu$m particle fraction.

1E. Composition:

| Lactose F.U. | 1000 g |
|---|---|
| Magnesium stearate | 10 g |
| Purified water | 405 g |

The lactose F.U. (1000 g) was suspended in the purified water (450 g) and the suspension heated to 70°C while stirring. The magnesium stearate (10 g) was added and mixed until homogeneously dispersed. The product was atomised, under suitable conditions, to obtain the required particle size. The product was then sifted through a 0.03 mm mesh eliminating the < 30$\mu$m particle fraction.

EXAMPLE 2

Pharmaceutical composition in powder form for the inhalation of beclomethasone dipropionate (BDP)

A composition to be used to fill 100,000 inhaler devices of the type mentioned above, each containing 100 unit doses of active principle for adults and children respectively.

|  | Adults (kg) | Children (kg) |
|---|---|---|
| Micronised beclomethasone dipropionate | 2 | 1 |
| Lactose and magnesium stearate conglomerate | 273 | 274 |

The active principle and the excipient were mixed thoroughly until homogeneous in a powder mixer. The mixture was divided and used to fill the inhaler devices by means of an automatic filling device. The mouthpiece was inserted on discharge from the filling device.

EXAMPLE 3

Pharmaceutical composition in powder form for the inhalation of salbutamol

A composition to be used to fill 100,000 inhaler devices of the type mentioned above, each containing 100 unit doses of active principle.

|  | kg |
|---|---|
| Micronised salbutamol | 4 |
| Lactose and magnesium stearate conglomerate | 271 |

The active principle and the excipient were mixed thoroughly until homogeneous in a powder mixer. The mixture was divided and used to fill the inhaler devices by means of an automatic filling device. The mouthpiece was inserted on discharge from the filling device.

EXAMPLE 4

Pharmaceutical composition in powder form for the inhalation of a combination of beclomethasone dipropionate (BDP) and salbutamol at a ratio of 1:2

A composition to be used to fill 100,000 inhaler devices of the type mentioned above, each containing 100 unit doses of active principle.

|  | kg |
|---|---|
| Micronised beclomethasone dipropionate | 2 |
| Micronised salbutamol | 4 |
| Lactose and magnesium stearate conglomerate | 269 |

The active principle and the excipients were mixed thoroughly until homogeneous in a powder mixer. The mixture was divided and used to fill the inhaler devices by means of an automatic filling device. The mouthpiece was inserted on discharge from the filling device. in order to check the properties of the pharmaceutical composition prepared using the new conglomerate and the operation of the inhaler designed for their administration, the following tests were carried out.

## TABLE 1

Comparison between two groups of devices for powder inhalation filled with 2 different pharmaceutical compositions formed by:

A. BDP + Lactose conglomerated with magnesium stearate

B. BDP + Commercially available microcrystalline lactose

Parameters tested: total weight of 4 metering operations; active principle content of the quantity of composition produced by 4 metering operation; ease of rotation of the apparatus.

| Compo-sition | Apparatus No. | Total weight of 4 distributions (in mg) | | | BDP content of 4 distributions (in mcg) | | | Ease of rotation (1) |
|---|---|---|---|---|---|---|---|---|
| | | min | max | diff(2) | min | max | diff(2) | |
| A | 1 | 109.8 | 117.2 | 7.4 | 765 | 827 | 62 | 1 |
| BDP + lac- | 2 | 107.1 | 115.9 | 8.8 | 782 | 823 | 41 | 2 |
| tose and Mg | 3 | 110.8 | 116.4 | 5.6 | 768 | 828 | 60 | 2 |
| stearate | 4 | 108.2 | 116.9 | 8.7 | 765 | 816 | 51 | 1 |
| agglomerate | 5 | 108.5 | 117.0 | 8.5 | 786 | 829 | 43 | 1 |
| | 6 | 108.8 | 116.0 | 7.2 | 781 | 831 | 50 | 1 |
| B | 1 | 93.8 | 102.1 | 8.3 | 712 | 852 | 140 | 3 |
| BDP + | 2 | 90.1 | 107.3 | 17.2 | 742 | 847 | 105 | 2 |
| commercial | 3 | 92.6 | 108.1 | 15.5 | 706 | 843 | 137 | 1 |
| microcry- | 4 | 91.8 | 106.2 | 14.4 | 723 | 816 | 93 | 2 |
| stalline | 5 | 90.5 | 103.8 | 13.3 | 715 | 838 | 123 | 2 |
| lactose | 6 | 95.5 | 108.5 | 13.0 | 697 | 831 | 134 | 4 |

7

1.      Arbitrary  evaluation  scale  with  values  between  1

         and 4 defined as follows:

         1 = easy;  2 = fairly easy; 3 = difficult; 4 = very

         difficult.

2.      Min = minimum value;  Max = maximum value;  Diff =

         difference BDP. = beclomethasone dipropionate.

The compositions prepared using the conglomerates described in Examples 1B-1E were evaluated in a similar way and provided comparable results.

A further important factor of evaluation which became evident during the tests was the fact that in the case of the conglomerate the residual quantity of powder in the container chamber of the inhaler required to enable a weight distribution within acceptable limits, was substantially lower that that required when using the simple microcrystalline excipient (approx. 300 mg as against approx. 500 mg).

The findings illustrated clearly show that the use of the new type of excipient for pharmaceutical formulations in the form of powders to be inhaled of the invention provides substantial advantages, significantly improving reproducibility and therefore the effectiveness of a widely used form of treatment which is also used for long periods.

**Claims**

1.  An excipient suitable for use in the preparation of pharmaceutical compositions in the form of powder for inhalation, constituted by microgranules between 30 and 150 $\mu$m in size of a conglomerate of one or more solid water-soluble diluents selected from lactose, xylitol, mannitol, arabinose, dextran and a mixture of lactose with 0.5 to 30% by weight of saccharose with a lubrificant selected from magnesium stearate, sodium benzoate, colloidal silica, hydrogenated oils.

2.  An excipient as claimed in claim 1, constituted by microgranules of a conglomerate of lactose with magnesium stearate.

3.  An excipient as claimed in claim 1, constituted by microgranules of a conglomerate of lactose with the addition of saccharose at a percentage of 4.5% and magnesium stearate.

4.  An excipient as claimed in claim 1, constituted by microgranules of a conglomerate of xylitol with sodium benzoate.

5.  A pharmaceutical composition in the form of micronized powder for inhalation, constituted by a mixture of a solid drug and an excipient as claimed in any one of claims 1 to 4.

6.  A pharmaceutical composition as claimed in claim 5, wherein the solid drug is crushed into particles between 0.1 and 10 $\mu$m in size.

7.  A pharmaceutical composition as claimed in claims 5 or 6, wherein the active principle is a drug for the treatment of bronchopulmonary disorders.

8.  A pharmaceutical composition as claimed in claims 5 to 7, wherein the active principle is an antihistamine, an antiallergic drug, a sympathomimetic amine, a steroid, an anticholinergic, a mucolytic or a mixture thereof.

9.  A pharmaceutical composition as claimed in any one of claims 5 to 8, wherein the active priciple is beclomethasone dipropionate, salbutamol, terbutaline, fenoterol, procaterol, pirbuterol, reproterol, clenbuterol, flunisolide, budesonide, sodium cromoglycate, ipratropium bromide, oxytropium bromide, ambroxol or a combination thereof.

**10.** A pharmaceutical composition as claimed in any one of claims 5 to 9, wherein the active principle is present at a ratio of between 0.01 and 99.5% by weight and the excipient at a ratio of between 99.9 and 0.5% by weight respectively.

**11.** A powder inhaler containing a pharmaceutical composition as defined in any one of claims 5 to 10.

**12.** A method for the preparation of an excipient as claimed in any one of claims 1 to 4, wherein the lubrificant is added to an aqueous solution of a portion of the solid diluent and the mixture obtaiend in this way is subsequently used for the granulation of the remainder of the solid diluent and is finally sifted through a 0.03 mm mesh to separate particles of less than 30 $\mu$m from the desired fraction.

**13.** A method as claimed in claim 12, wherein the conglomerate is obtained by granulation in a fluidised bed or by atomisation.

**14.** A method for the preparation of pharmaceutical compositions as claimed in any one of claims 5 to 10, wherein a solid drug crushed into particles between 0.1 and 10 $\mu$m in size is mixed, in a suitable powder mixer, with an excipient of the type defined in any one of claims 1 to 7 and formed by particles between 30 and 150 $\mu$m in size.

**Patentansprüche**

**1.** Excipiens, geeignet zur Verwendung bei der Herstellung von pharmazeutischen Präparaten in Form eines Pulvers zur Inhalation, bestehend aus Mikrokörnchen mit einer Größe zwischen 30 und 150 $\mu$m eines Konglomerats aus einem oder mehreren festen wasserlöslichen Verdünnungsmitteln, ausgewählt aus Lactose, Xylit, Mannit, Arabinose, Dextran und einem Gemisch aus Lactose mit 0,5 bis 30 Gew.-% Saccharose und einem Gleitmittel, ausgewählt aus Magnesiumstearat, Natriumbenzoat, kolloidaler Kieselsäure, hydrierten Ölen.

**2.** Excipiens nach Anspruch 1, dadurch **gekennzeichnet,** daß es aus Mikrokörnchen eines Konglomerats aus Lactose mit Magnesiumstearat besteht.

**3.** Excipiens nach Anspruch 1, dadurch **gekennzeichnet,** daß es aus Mikrokörnchen eines Konglomerats aus Lactose unter Zusatz von Saccharose in einem Prozentsatz von 4,5 % und Magnesiumstearat besteht.

**4.** Excipiens nach Anspruch 1, dadurch **gekennzeichnet,** daß es aus Mikrokörnchen eines Konglomerats aus Xylit mit Natriumbenzoat besteht.

**5.** Pharmazeutisches Präparat in Form eines mikronisierten Pulvers zur Inhalation, dadurch **gekennzeichnet,** daß es aus einem Gemisch aus einem festen Arzneimittel und einem Excipiens nach einem der Ansprüche 1 bis 4 besteht.

**6.** Pharmazeutisches Präparat nach Anspruch 5, dadurch **gekennzeichnet,** daß das feste Arzneimittel zu 0,1 bis 10 $\mu$m großen Teilchen vermahlen ist.

**7.** Pharmazeutisches Präparat nach den Ansprüchen 5 oder 6, dadurch **gekennzeichnet,** daß der Wirkstoff ein Arzneimittel für die Behandlung von bronchopulmonalen Störungen ist.

**8.** Pharmazeutisches Präparat nach den Ansprüchen 5 bis 7, dadurch **gekennzeichnet,** daß der Wirkstoff ein Antihistamin, ein Antiallergikum, ein sympathomimetisches Amin, ein Steroid, ein Anticholinergikum, ein Mucolytikum oder ein Gemisch davon ist.

**9.** Pharmazeutisches Präparat nach einem der Ansprüche 5 bis 8, dadurch **gekennzeichnet,** daß der Wirkstoff Beclomethasondipropionat, Salbutamol, Terbutalin, Fenoterol, Procaterol, Pirbuterol, Reproterol, Clenbuterol, Flunisolid, Budesonid, Natriumcromoglycat, Ipratropiumbromid, Oxytropiumbromid, Ambroxol oder eine Kombination davon ist.

**10.** Pharmazeutisches Präparat nach einem der Ansprüche 5 bis 9, dadurch **gekennzeichnet,** daß der Wirkstoff in einem Anteil von 0,01 bis 99,5 Gew.-%, und das Excipiens in einem Anteil von 99,5 bis 0,5 Gew.-% vorhanden sind.

**11.** Pulverinhalator, enthaltend ein pharmazeutisches Präparat nach einem der Ansprüche 5 bis 10.

**12.** Verfahren zur Herstellung eines Excipiens nach einem der Ansprüche 1 bis 4, dadurch **gekennzeichnet,** daß das Gleitmittel zu einer wäßrigen Lösung eines Teils des festen Verdünnungsmittels gegeben wird, und das so erhaltene Gemisch anschließend zum Granulieren des restlichen festen Verdünnungsmittels verwendet wird, und dieses schließlich durch ein 0,03-mm-Sieb gesiebt wird, um Teilchen, die kleiner als 30 $\mu$m sind, von der gewünschten Fraktion abzutrennen.

**13.** Verfahren nach Anspruch 12, dadurch **gekennzeichnet,** daß das Konglomerat durch Granulieren in einem Wirbelbett oder durch Zerstäuben erhalten wird.

**14.** Verfahren zur Herstellung von pharmazeutischen Präparaten nach einem der Ansprüche 5 bis 10, dadurch **gekennzeichnet,** daß ein festes Arzneimittel, das zu 0,1 bis 10 $\mu$m großen Teilchen vermahlen ist, in einem geeigneten Pulvermischer mit einem Excipiens nach einem der Ansprüche 1 bis 7 aus 30 bis 150 $\mu$m großen Teilchen vermischt wird.

**Revendications**

**1.** Excipient convenant à l'utilisation dans la préparation de compositions pharmaceutiques sous forme de poudre pour inhalation, constitué de microgranules, dont la taille est comprise entre 30 et 150 $\mu$m, d'un conglomérat de un ou plusieurs diluants solides hydrosolubles choisi parmi le lactose, le xylitol, le mannitol, l'arabinose, le dextrane et un mélange de lactose et de 0,5 à 30 % en poids de saccharose, avec un lubrifiant choisi parmi le stéarate de magnésium, le benzoate de sodium, la silice colloïdale, les huiles hydrogénées.

**2.** Un excipient tel que revendiqué dans la revendication 1, constitué de microgranules d'un conglomérat de lactose avec du stéarate de magnésium.

**3.** Excipient tel que revendiqué dans la revendication 1, constitué de microgranules d'un conglomérat de lactose avec l'addition de saccharose à 4,5 % et de stéarate de magnésium.

**4.** Un excipient tel que revendiqué dans la revendication 1, constitué de microgranules d'un conglomérat de xylitol avec du benzoate de sodium.

**5.** Composition pharmaceutique sous forme de poudre micronisée pour inhalation, constituée d'un mélange d'un médicament solide et d'un excipient tel que revendiqué dans l'une quelconque des revendications 1 à 4.

**6.** Composition pharmaceutique telle que revendiquée dans la revendication 5, dans laquelle le médicament solide est broyé en particules de taille comprise entre 0,1 et 10 $\mu$m.

**7.** Composition pharmaceutique telle que revendiquée dans les revendications 5 ou 6, dans lesquelles le principe actif est un médicament pour le traitement des troubles broncho-pulmonaires.

**8.** Composition pharmaceutique telle que revendiquée dans les revendications 5 à 7, dans laquelle le principe actif est une antihistamine, un medicament antiallergique, un amine sympathomimétique, un stéroïde, un anticholinergique, un mucolytique ou un mélange de ceux-ci.

**9.** Composition pharmaceutique telle que revendiquée dans l'une quelconque des revendications 5 à 8, dans laquelle le principe actif est le dipropionate de béclométhasone, le salbutamol, la terbutaline, le fénotérol, le procatérol, le pirbutérol, le reprotérol, le clenbutérol, le flunisolide, le budésonide, le cromoglycate de sodium, le bromure d'ipratropium, le bromure d'oxytropium, l'ambroxol ou une combinaison de ceux-ci.

**10.** Composition pharmaceutique telle que revendiquée dans l'une quelconque des revendications 5 à 9, dans laquelle le principe actif est présent selon une proportion comprise entre 0,01 et 99,5 % en poids et l'excipient selon une proportion comprise entre 99,9 et 0,5 % en poids, respectivement.

**11.** Inhalateur à poudre contenant une composition pharmaceutique telle que définie dans l'une quelconque des revendications 5 à 10.

**12.** Procédé de préparation d'un excipient tel que revendiqué dans l'une quelconque des revendications 1 à 4, dans lequel le lubrifiant est ajouté à une solution aqueuse d'une portion du diluant solide et dans lequel le mélange ainsi obtenu est ensuite utilisé pour la granulation du reste du diluant solide, et est finalement tamisé par un tamis d'ouverture de maille de 0,03 mm pour séparer les particules de taille inférieure à 30 $\mu$m de la fraction désirée.

**13.** Procédé tel que revendiqué dans la revendication 12, dans lequel le conglomérat est obtenu par granulation sur lit fluidisé ou par atomisation.

**14.** Procédé de préparation de compositions pharmaceutiques telles que revendiquées dans l'une quelconque des revendications 5 à 10, dans lequel un médicament solide broyé en particules de taille comprise entre 0,1 et 10 $\mu$m est mélangé, dans un mélangeur à poudre approprié, avec un excipient du type défini dans l'une quelconque des revendications 1 à 7 et formé en particules de taille comprise entre 30 et 150 $\mu$m.